# EUROPEAN PATENT APPLICATION

(11) **EP 4 473 897 A1**
(43) Date of publication of application: **11.12.2024**
(21) Application number: 23749557.7
(22) Date of filing: 20.01.2023
(51) Int. Cl.: A61B 3/04, A61B 3/09, G02C 5/20, G02C 13/00

(54) **LENS FRAME FOR EYE EXAMINATION, MEASUREMENT METHOD FOR NUMERICAL VALUE RELATED TO EYESIGHT, AND SPECTACLE LENS DESIGN METHOD**

(30) Priority: 04.02.2022 JP 2022016281
(71) Applicant: HOYA LENS THAILAND LTD., Pathumthani 12130 (TH)
(72) Inventor: YAMAGUCHI, Eiichiro, Tokyo 160-8347 (JP); SONEHARA, Toshiaki, Tokyo 160-8347 (JP); ITO, Ayumu, Tokyo 160-8347 (JP)
(74) Representative: Betten & Resch
(86) International application number: PCT/JP2023/001756
(87) International publication number: WO 2023/149241

(57) **Abstract**

A lens frame for eye examination with which performing an eye examination can be performed in an appropriate state even in a state such as a downward view other than a horizontal view is provided.

A lens frame 1 includes a lens holding member 20 having one pair of lens housings 220 holding lenses and one pair of temple members 10, and the temple member 10 includes each front temple part 32 supporting the lens holding member 20, a rear temple part 34 connected to a rear side of the front temple part 32, and an angle adjustable connection part 36 that is able to adjust an angle of the front temple part 32 with respect to the rear temple part 34 using a rotation center portion as a center, and the front temple part 32 has a length adjusting mechanism 324 that is able to adjust a length of the front temple part 32.

## Description

### [Technical Field]

The present disclosure relates to a lens frame for an eye examination, a method for measuring numerical values relating to eyesight, and a method for designing spectacle lenses.

### [Background Art]

Typically, in order to create a prescription necessary for making eyeglasses, a method in which a subject subjectively tests a refractive index while wearing a lens frame for eye examination is used. The lens frame for eye examination has a lens holding frame, a plurality of lenses for eye examination having different characteristics are sequentially mounted in this lens holding frame, and an appropriate eyeglass prescription for the subject is determined.

As such a lens frame for eye examination, for example, in PTL1, a lens frame including one pair of lens holding devices and temples attached to the respective lens holding devices in which a rotating means used for adjusting the inclination of a temple end part is disposed between the lens holding device and the temple end part is disclosed.

### [Citation List]

### [Patent Literature]

[PTL 1] Japanese Patent Application Publication No. 2017-104544

### [Summary of Invention]

### [Technical Problem]

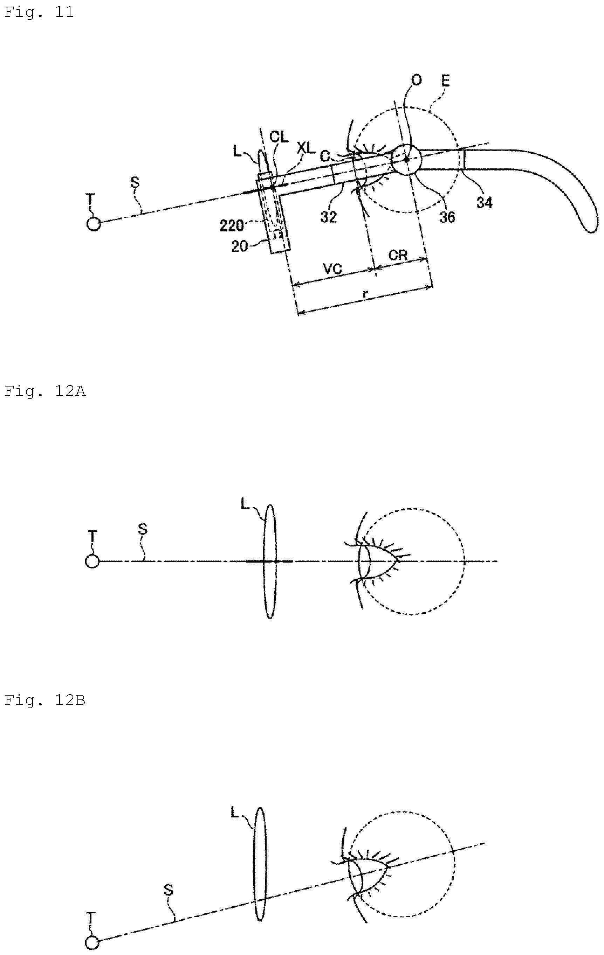

Here, when an eye examination is performed using a lens frame for eye examination, as illustrated in Fig. 12A, in a case in which visual acuity is measured in a state in which a visual target T is viewed through an eye examination lens L in a horizontal view (viewed in a horizontal direction), a line of sight S passes through the optical center of the lens in parallel with the optical axis of the eye examination lens L, and thus visual acuity can be accurately measured.

However, as illustrated in Fig. 12B, in a state in which a subject views a visual target T through an eye examination lens L in a downward view (a state in which a lower side with respect to a horizontal direction is viewed), there are cases in which visual acuity cannot be accurately measured. The reason for this is that, when the downward view is performed, in accordance with the line of sight S not passing through the optical center of the eye examination lens L and occurrence of an axial deviation, blurring occurs due to aberration and a diopter error of the eye examination lens L, a distance between the eye examination lens L and a corneal apex is changed in a horizontal view and a downward view, or an unintentional prism is added. During an eye examination, although such use of the line of sight is rarely instructed, particularly when a user using a progressive power lens views a near place, such a downward view is routinely necessary. For this reason, although there is a potential need for accurately measuring refractive power in the state of such a downward view, it has been difficult to measure accurate refractive power using a conventional lens frame for eye examination.

The present disclosure is in view of the problems described above, and an object thereof is to provide a lens frame for eye examination, with which eye examination can be performed in an appropriate state even in a state of a downward view or the like other than a horizontal view. In this specification, an eye examination is a method of measuring numerical values relating to eyesight and represents a series of operations of a subject visually recognizing a target and acquiring a refractive index value, a prism value, and the like in accordance with a subjective response thereof.

### [Solution to Problem]

According to the present disclosure, there is provided a lens frame for eye examination including a lens holding member having one pair of lens housings holding lenses and one pair of temples, and the temples each include a front temple part supporting the lens holding member, a rear temple part connected to a rear side of the front temple part, and an angle adjusting mechanism that is able to adjust an angle of the front temple part with respect to the rear temple part using a rotation center portion as a center, and the front temple part has a length adjusting mechanism that is able to adjust a length of the front temple part.

### [Advantageous Effects of Invention]

According to the present invention, a lens frame for eye examination, with which eye examination can be performed in an appropriate state even in a state of a downward view or the like other than a horizontal view, is provided.

### [Brief Description of Drawings]

[Fig. 1A]

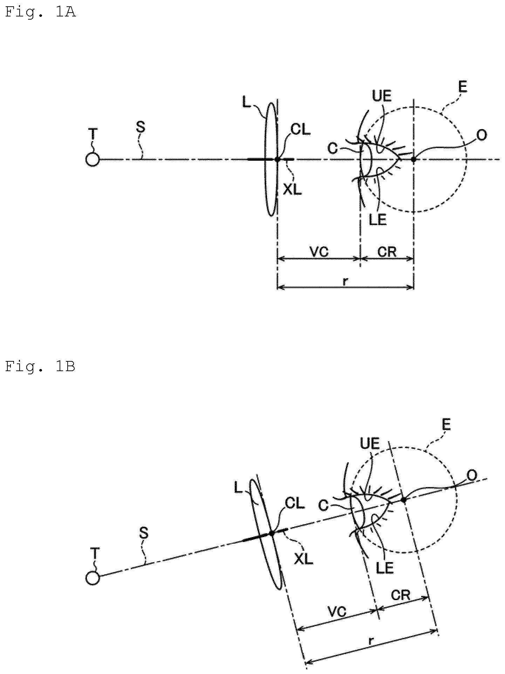

   Fig. 1A is a diagram illustrating a state in which eye examination is performed in a horizontal view in a method of measuring numerical values relating to eyesight proposed by the inventors and the like.
[Fig. 1B]
   Fig. 1B is a diagram illustrating a state in which an eye examination is performed in a downward view in the method of measuring numerical values relating to eyesight proposed by the inventors and the like.
[Fig. 2]

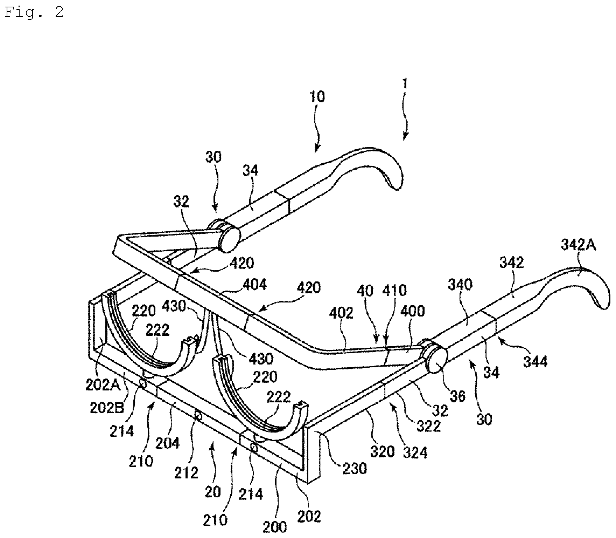

   Fig. 2 is a perspective view illustrating a lens frame according to one embodiment of the present invention.
[Fig. 3]

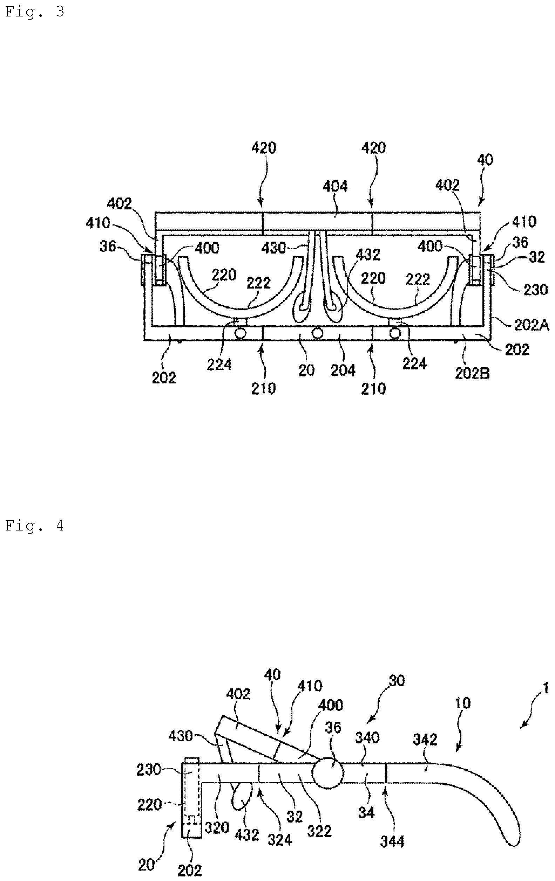

   Fig. 3 is a front view illustrating a lens frame according to one embodiment of the present invention.
[Fig. 4]
   Fig. 4 is a side view illustrating a lens frame according to one embodiment of the present invention.
[Fig. 5]

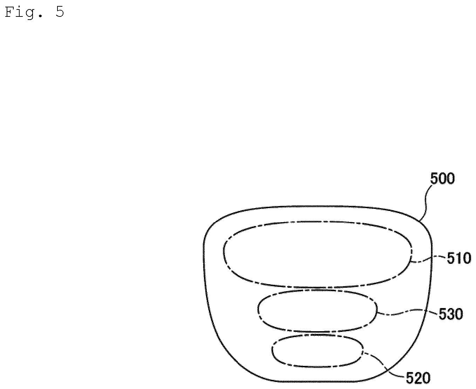

   Fig. 5 is a front view illustrating a progressive power lens manufactured using a measurement method and a design method for numerical values relating to eyesight according to one embodiment of the present invention.
[Fig. 6]

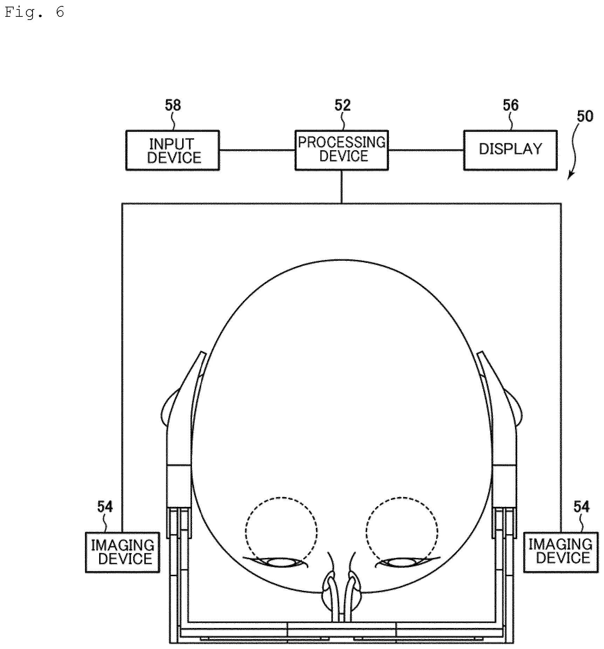

   Fig. 6 is a front view illustrating a progressive power lens manufactured using a measurement method and a design method for numerical values relating to eyesight according to this embodiment.
[Fig. 7]

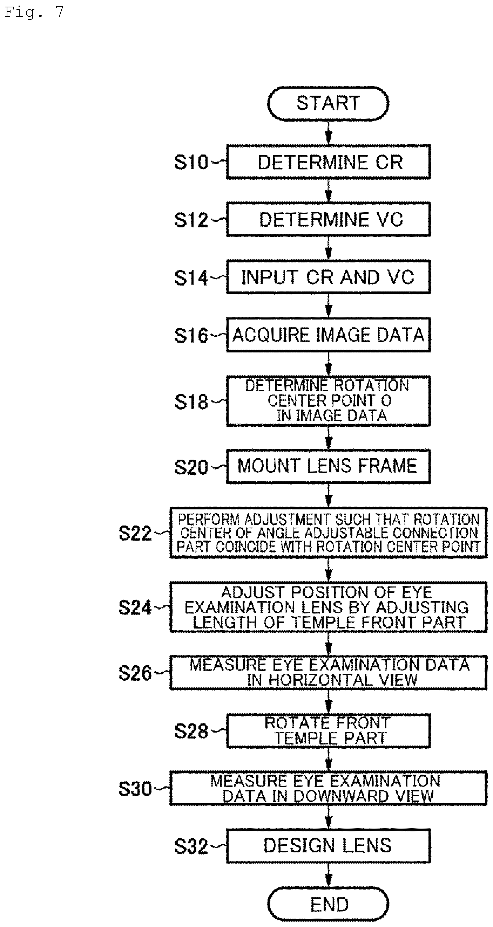

   Fig. 7 is a flowchart illustrating the flow of a method of designing a spectacle lens on the basis of a method of measuring numerical values relating to eyesight using a lens frame according to one embodiment of the present invention and a result of the measurement.
[Fig. 8]

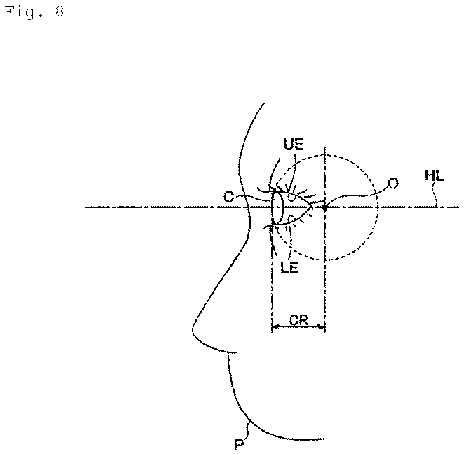

   Fig. 8 is a diagram illustrating one example of image data captured by an imaging device.
[Fig. 9]

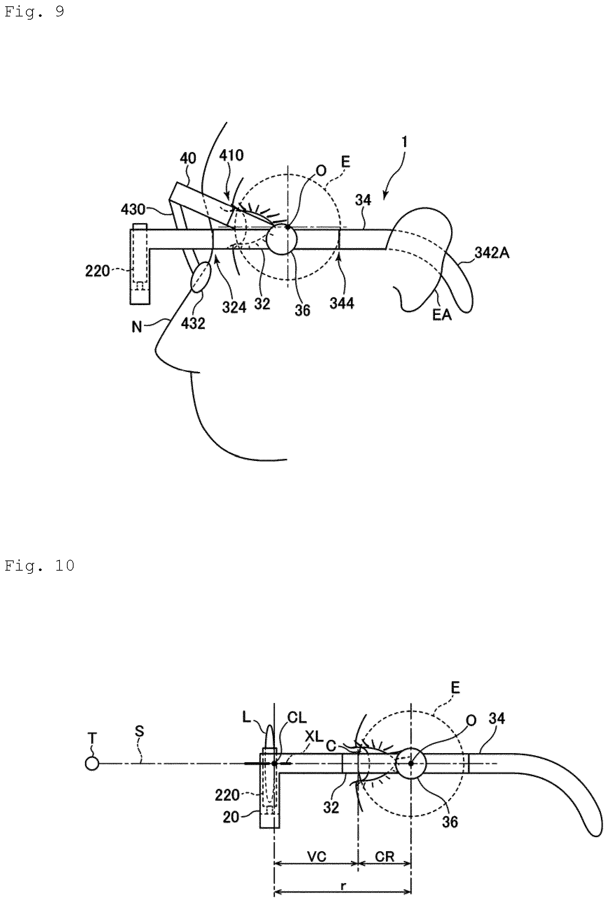

   Fig. 9 is a side view illustrating a state in which a lens frame according to one embodiment of the present invention is worn by a subject.
[Fig. 10]
   Fig. 10 is a diagram illustrating a view in which the lens frame is adjusted such that a rotation center portion of an angle adjustable connection part coincides with a rotation center point.
[Fig. 11]
   Fig. 11 is a diagram illustrating a view in which a front temple part is rotated to a lower side by an angle adjustable connection part.
[Fig. 12A]
   Fig. 12 is a diagram illustrating a view in which visual acuity is measured in a state in which a visual target is seen through an eye examination lens in a horizontal view in a conventional technology.
[Fig. 12B]
   Fig. 12B is a diagram illustrating a view in which visual acuity is measured in a state in which a visual target is seen through an eye examination lens in a downward view in a conventional technology.

### [Description of Embodiments]

As a result of thorough review taking the problems described above into account, the inventors and the like thought that accurate eye examination can be performed using a procedure as below.

Figs. 1A and 1B are diagrams illustrating states in which eye examination is performed in a horizontal view and a downward view in a method of measuring numerical values relating to eyesight proposed by the inventors and the like. An eyeball E can be regarded as an approximate sphere, and at the time of transition from the horizontal view to the downward view, the eyeball E rotates such that the line of sight points downward. At this time, the eyeball E can be regarded as rotating around a rotation center point 0, which is a center point of the sphere in the state in which the eyeball E is regarded as a sphere, as the center.

This rotation center point O is located on a horizontal line passing through the center of a circle at the time of approximating a cross-sectional shape of the eyeball surface as an arc or near the horizontal line. When ptosis and the like caused by aging or the like are not severe, for the convenience of description, the rotation center point may be regarded to be located on a horizontal line passing through a point that vertically bisects a palpebral fissure height (interpalpebral fissure height) in the horizontal view or near the horizontal line. The palpebral fissure height is a distance from a lower eyelid edge LE to an upper eyelid edge UE in a vertical direction.

As illustrated in Fig. 1A, in order to perform an eye examination in a horizontal view, it is performed in a state in which the line of sight coincides with an optical axis XL of the eye examination lens, the line of sight passes through the optical axis XL of the eye examination lens and a reference point CL on a rear face of the lens, and the eye examination lens is held at a position for which a distance from the reference point CL to the corneal apex is a predetermined distance VC.

As illustrated in Fig. 1B, in order to perform an eye examination in a downward view, a subject directs his or her line of sight S at a predetermined angle below the horizontal direction. At this time, the eye examination lens L is rotated by the same angle as the rotation angle of the eyeball E around the rotation center point O as its center while a distance r between the reference point CL on the rear face of the eye examination lens and the rotation center point O is maintained. This distance r can be calculated as a sum of the value of the distance VC from the reference point CL on the rear face of the eye examination lens to the apex of the corneal C of the eyeball E of the subject and the distance CR from the apex of the corneal C to the rotation center point O of the eyeball E. In accordance with this, the distance from the reference point CL on the rear face of the eye examination lens L to the apex of the corneal C becomes the same as the distance VC in the horizontal view. In addition, the line of sight S coincides with the optical axis XL of the eye examination lens L.

By performing an eye examination in the downward view in this way, the line of sight S does not have an occurrence of an optical center axis deviation of the eye examination lens L, and an eye examination having high accuracy can be performed without blurring or a prism caused by aberrations.

Hereinafter, a lens frame for eye examination according to one embodiment of the present invention will be described with reference to the drawings. The lens frame according to this embodiment is for realizing a method of measuring numerical values relating to eyesight devised by the inventors and the like described above.

Figs. 2 to 4 illustrate a lens frame according to one embodiment of the present invention, Fig. 2 is a perspective view, Fig. 3 is a front view, and Fig. 4 is a side view. As illustrated in Figs. 2 to 4, the lens frame 1 according to this embodiment includes a frame main body 10 and a lens holding member 20.

The frame main body 10 has one pair of temple members 30 and a nose rest frame 40.

Each of the temple members 30 includes a front temple part 32, a rear temple part 34, and an angle adjustable connection part 36 that connects the front temple part 32 to the rear temple part 34 to be able to rotate with respect thereto.

The rear temple part 34 has a shape in which a front part linearly extends to front and rear sides, and a rear end part curves toward the lower side. The rear temple part 34 includes a first rear temple member 340 positioned on the front side and a second rear temple member 342 positioned on the rear side.

The first rear temple member 340 has a hollow rectangular cross-section and extends linearly. The second rear temple member 342 has a hollow rectangular cross-section, and a curved part 342A that curves down is formed at a rear end part. The curved part 342A of this second rear temple member 342 functions as an ear pad when a subject wears the lens frame 1.

A width and a height of the inner face of the cross-section of the second rear temple member 342 are approximately the same as a width and a height of the outer face of the cross-section of the first rear temple member 340. A rear end part of the first rear temple member 340 is housed in a nested form inside of a front end part of the second rear temple member 342. By using such a nested structure, the second rear temple member 342 can be moved forward/backward with respect to the first rear temple member 340. In other words, this nested structure functions as a length adjusting mechanism 344 used for adjusting the length of the rear temple part 34. The configuration of the length adjusting mechanism of the rear temple part is not limited to the nested structure, and for example, an appropriate configuration capable of adjusting and maintaining the length such as a configuration in which a slit is formed in one of the first rear temple member 340 and the second rear temple member 342, a protrusion part is formed in the second rear temple member 342, and this protrusion part can slide inside of the slit may be employed. In addition, the cross-sectional shape of the first rear temple member 340 and the second rear temple member 342 is not limited to a rectangle, and an appropriate shape such as a circular shape can be employed.

The front temple part 32 has a shape that extends linearly to the front side. The front temple part 32 includes a first front temple member 320 positioned on the front side and a second front temple member 322 positioned on the rear side.

The first front temple member 320 has a hollow rectangular cross-section and extends linearly.

The second front temple member 322 has a hollow rectangular cross-section and extends linearly. A width and a height of the inner face of the cross-section of the second front temple member 322 are approximately the same as a width and a height of the outer face of the cross-section of the second front temple member 322. The rear end part of the first front temple member 320 is housed in a nested form inside of the front end part of the second front temple member 322. By using such a nested structure, the second front temple member 322 can be moved forward/backward with respect to the first front temple member 320. In other words, this nested structure functions as a length adjusting mechanism 324 used for adjusting the length of the front temple part 32. In addition, similar to the configuration of the length adjusting mechanism 344 of the rear temple part 34, also as the configuration of the length adjusting mechanism 324 of the front temple part 32, an appropriate configuration can be applied. Furthermore, the cross-sectional shape of the first front temple member 320 and the second front temple member 322 is not limited to a rectangle, and an appropriate shape such as a circular shape can be applied.

The angle adjustable connection part 36 rotatably connects the front temple part 32 and the nose rest frame 40 to the rear temple part 34. In other words, a rotation center portion at which the front temple part 32 and the nose rest frame 40 rotate with respect to the rear temple part 34 is positioned at the center of the angle adjustable connection part 36. The angle adjustable connection part 36 can rotate the front temple part 32 and the nose rest frame 40 in a plane defined in the vertical direction and the forward/backward direction with respect to the rear temple part 34 and can maintain an angle of each of the front temple part 32 and the nose rest frame 40 with respect to the rear temple part 34 at a desirable angle. As a mechanism of such an angle adjustable connection part 36, a rachet mechanism or the like can be employed. The mechanism of the angle adjustable connection part 36 is not limited to a configuration that is able to change the angle in a stepped manner such as the rachet mechanism but may be a configuration that is able to change the angle continuously.

The front temple part 32 can be rotated in a direction in which a front side is inclined to the upper side or the lower side from a state of being in parallel with the rear temple part 34. An angle range in which the front temple part 32 can rotate is preferably 60 degrees in each of upward/downward directions and is more preferably 30 degrees in each of the upward/downward directions.

The nose rest frame 40 has one pair of first frame members 400 extending to the front side in the horizontal view from each angle adjustable connection part 36, one pair of second bending frame members 402 connected to the front side of the first frame members 400, and a third horizontal frame member 404 passing over a space between the one pair of second bending frame members.

A rear end of the first frame member 400 is connected to the angle adjustable connection part 36. The first frame member 400 has a hollow rectangular cross-section and extends linearly.

The second bending frame member 402 has a hollow rectangular cross-section and vertically bends between a base end part and a tip end part. A width and a height of the inner face of the cross-section of the second bending frame member 402 are approximately the same as a width and a height of the outer face of the cross-section of the first frame member 400. A front end part of the first frame member 400 is housed in a nested form inside of a rear end part of the second bending frame member 402. By using such a nested structure, the second bending frame member 402 can be moved forward and backward with respect to the first frame member 400. In other words, this nested structure functions as a length adjusting mechanism 410 used for adjusting and maintaining the length of the nose rest frame 40 in a forward/backward direction.

The third horizontal frame member 404 has a hollow rectangular cross-section and extends linearly in the horizontal direction. A width and a height of the outer face of the cross-section of the third horizontal frame member 404 are approximately the same as a width and a height of the inner face of the cross-section of the second bending frame member 402. Both ends of the third horizontal frame member 404 are housed in a nested form inside of the tip end of the second bending frame member 402. By using such a nested structure, each second bending frame member 402 can be relatively moved in the horizontal direction with respect to the third horizontal frame member 404, in other words, can freely change a horizontal-direction width of the nose rest frame 40. In other words, this nested structure functions as a width adjusting mechanism 420 used for adjusting the horizontal-direction width of the nose rest frame 40.

At the horizontal-direction center of the nose rest frame 40, one pair of nose rest supporting parts 430 are mounted with horizontal symmetry. The nose rest supporting part 430 is mounted to extend downward from the center portion of the nose rest frame 40, and a nose pad 432 is mounted at the tip end. When the lens frame 1 is mounted, the nose rests 432 of the nose pad supporting part 430 are arranged to come into contact with both side faces of the nose of a subject. The nose rest supporting part 430 may be composed of an elastic material such as rubber or may be composed of a deformable resin.

The lens holding member 20 includes a bridge member passing over a space between the front temple parts 32, a connection part 230 connecting both ends of the bridge member 200 to the front end parts of the front temple parts 32, and one pair of lens housings 220 mounted in the bridge member 200.

The bridge member 200 has one pair of first bending members 202 and a second horizontal member 204 disposed between the first bending members 202.

The first bending member 202 has a vertical part 202A of which an upper end extends with being connected to the front end of the front temple part 32 through the connection part 230 and a horizontal part 202B that vertically bends from the vertical part 202A and extends to the inner side in the horizontal direction. The first bending member 202 has a hollow rectangular cross-section.

The second horizontal member 204 has a hollow rectangular cross-section and extends linearly in the horizontal direction. A width and a height of the outer face of the cross-section of the second horizontal member 204 are approximately the same as a width and a height of the inner face of the cross-section of the first bending member 202. Both ends of the second horizontal member 204 is housed in a nested form inside of the tip end of the first bending member 202. By using such a nested structure, each first bending member 202 can be relatively moved in the horizontal direction with respect to the second horizontal member 204, in other words, can freely change a horizontal-direction width of the bridge member 200. In other words, this nested structure functions as a width adjusting mechanism 210 used for adjusting and maintaining the horizontal-direction width of the bridge member 200.

The connection part 230 connects the front end of the front temple part 32 and an upper end of the vertical part 202A of the first bending member 202 of the bridge member 200. Although the connection part 230 can be set to an arbitrary height in accordance with a length of the vertical part 202A of the first bending member 202 of the bridge member 200, the connection part 230 is positioned at the same height as that of the center of the eye examination lens mounted in the lens housing 220 in this embodiment. In accordance with this, change to a predetermined length using the length adjusting mechanism 324 of the front temple part 32 to be described below can be performed more smoothly.

Each lens housing 220 includes a lens housing main body 222 and a pillar part 224 supporting the lens housing main body 222. The lens housing main body 222 is an arc-shaped member having rigidity capable of holding an eye examination lens, and on an upper face thereof, grooves used for mounting a maximum of three eye examination lenses such as a trial lens, an eye examination lens, and the like are formed.

The pillar part 224 has a cylindrical shape and extends downward from a lower end of the lens housing 220. The lens housing 220 is mounted on the upper face of the horizontal part 202B of the first bending member 202 of the bridge member 200. In the bridge member 200, a lens gap adjusting mechanism 212 enabling the lens housing 220 to move in the horizontal direction along the horizontal part 202B is built, and a bending angle adjusting mechanism 214 enabling the lens housing 220 to rotate around the center axis of the pillar part 224 is built.

As the lens gap adjusting mechanism 212 and the bending angle adjusting mechanism 214, for example, a configuration in which a slit is formed in the horizontal part 202B, and a base portion of the pillar part 224 is inserted inside of the slit in a rotatable state or the like can be employed, and, by rotating dial members 212 and 214, a gap of the lens housing 220 (a gap between the eye examination lens) and an angle of the lens housing 220 (an angle of the eye examination lens) can be changed.

In addition, horizontal-direction movement of the lens housings 220 according to the lens gap adjusting mechanism 212 is preferably configured to be movement of one pair of the lens housings 220 with horizontal symmetry. As such a configuration, a configuration in which screw rods in which screw grooves are formed with horizontal symmetry are arranged inside of the first bending member 202 and the second horizontal part, nuts that are screwed to these screw rods are mounted in a lower end part of the pillar part 224, and the screw rods are rotated or the like can be employed.

Hereinafter, a method of measuring numerical values relating to eyesight using the lens frame 1 described above and a method of designing spectacle lenses on the basis of a result of the measurement will be described.

In the following description, a case in which eye examination for manufacturing progressive power lenses is performed will be described. Fig. 5 is a front view illustrating a progressive power lens manufactured using the method of measuring numerical values relating to eyesight and the design method according to this embodiment. As illustrated in Fig. 5, the progressive power lens 500 includes a near portion 520 used for viewing a near distance that is positioned on a lower side, a distance portion 510 used for viewing a far distance longer than the near distance that is positioned over the center from the upper side, and a middle portion 530 that is positioned between the near portion 520 and the distance portion 510 and has a progressive refraction function in which the lens power continuously changes.

The method of measuring numerical values relating to eyesight according to this embodiment is performed using a frame position adjusting system 50 illustrated in Fig. 6.

As illustrated in Fig. 6, the frame position adjusting system 50 includes a processing device 52, one pair of imaging devices 54 connected to the processing device 52 to be able to communicate therewith, a display 56 connected to the processing device 52 to be able to communicate therewith, and an input device 58 connected to the processing device 52 to be able to communicate therewith.

One pair of imaging devices 54 are arranged on both sides of the head of a subject to have the head of the subject interposed therebetween. The imaging device 54, for example, is a digital camera and images the head of the subject near the eyeball from right beside. Image data imaged by the imaging device 54 is sequentially transmitted to the processing device 52.

The processing device 52, for example, is a device such as a personal computer including a CPU, a memory, a recording medium, an input interface, and an output interface. The processing device 52 reads a program recorded on the recording medium into the memory, and the CPU executes the program, whereby, as will be described in detail below, a rotation center of the eyeball of the subject is identified on the basis of the image data received from the imaging device 54. Then, the processing device 52 outputs image data acquired by superimposing the position of the rotation center onto an image captured by the imaging device 54 to the display 56.

The display 56 displays the image data received from the processing device 52 as an image.

The input device 58, for example, is a keyboard or a touch panel and receives inputs of CR and VC determined as will be described below.

Fig. 7 is a flowchart illustrating the flow of the method of measuring numerical values relating to eyesight using the lens frame according to this embodiment and the method of designing spectacle lenses on the basis of a result of the measurement.

As illustrated in Fig. 7, first, a distance CR from the corneal apex of a subject to the rotation center point is determined (S10). As the method for determining CR, for example, a known method such as a method disclosed in Japanese Patent Application Publication No. 2011-39552 can be employed. In other words, a method using an eyeball rotation point measuring device, a method of acquiring CR using an arithmetic operation from intersections of line of sights of different directions, or the like can be employed.

In addition, as a practical method that is a simplified method, an ocular axial length measuring device that is widely used generally can be used. More specifically, there are a method in which an ocular axial length is measured, and an eyeball rotation point is calculated from the measured ocular axial length and the like. In this method, by using general statistical data of a relative position of an eyeball rotation point with respect to an actually-measured ocular axial length, a relative position coefficient of a distance from the corneal apex to the rotation center point with respect to the ocular axial length is calculated in advance. For example, when an ocular axial length is 24 millimeters, and the distance CR from the corneal apex to the rotation center point is 13 millimeters as average data, 13/24 = 0.54 becomes the relative position coefficient used for calculation. Thus, in a case in which the ocular axial length of a subject is detected as being 27 millimeters by the ocular axial length measuring device, by using this relative position coefficient 0.54, the value of CR of this subject is set as 27 millimeters x 0.54 = 14.6 millimeters. In addition, other than that, by using various methods, a correlation between an ocular axial length and CR is identified, and the distance CR from the corneal apex to the rotation center point may be set on the basis of this correlation and an actually-measured ocular axial length.

In addition, as the value of CR, data of an average value is recorded in advance, and this average value may be used. For example, data of an average value of CR is recorded with being classified into an age, a height, and the like, an average value of CR is acquired from this data on the basis of the age, the height, and the like of a subject, and the value may be used as the value of CR of the subject.

Next, a distance VC from a reference point on the rear face of the spectacle lens to the corneal apex of the eyeball of a spectacle wearing person at the time of the subject being wearing the spectacle is determined (S12). This distance VC is also referred to as an inter-corneal apex distance (CVD). As a method for calculating this distance VC, for example, a method described in Japanese Patent Application Publication No. 2016-167038 or WO 2014/133166 can be employed.

Then, an eye examiner inputs CR and VC determined in this way to the input device 58 of the frame position adjusting system 50 to the input device 58 (S14). Data relating to CR and VC of which input has been received by the input device 58 is transmitted to the processing device 52. In addition, the step of determining these CR and VC may be performed on the basis of image data acquired by the imaging device 54 using the frame position adjusting system 50. In this case, it is preferable that an imaging device be disposed also in front of the subject.

Next, a subject is seated such that one pair of imaging devices 54 are positioned on both sides of his or her face to take a horizontal view and starts up the frame position adjusting system 50. When the frame position adjusting system 50 is started up, the processing device 52 acquires image data imaged by the imaging device 54 (S16). Fig. 8 is a diagram illustrating one example of image data imaged by the imaging device. When a side face of a face of a subject is detected in the image data imaged by the imaging device 54, the processing device 52 of the frame position adjusting system 50 determines a rotation center point O in the image data (S18). More specifically, first, as illustrated in Fig. 8, areas of an upper eyelid edge UE, a lower eyelid edge LE, and an eyeball surface C in the image data are determined through image processing. Then, a horizontal line HL bisecting the palpebral fissure height (interpalpebral fissure height) of the subject, that is, a horizontal line passing through a middle point between the apex of the upper eyelid edge UE and the lower point of the lower eyelid edge LE is determined through image processing. Then, a point that is positioned to a rear side of the apex of the corneal C on this horizontal line HL by the distance CR is determined as a rotation center point O of the subject. In addition, this horizontal line HL is not limited to the horizontal line bisecting the palpebral fissure, and by setting this horizontal line as a horizontal line passing through the center of a circle at the time of regarding the shape of the eyeball surface on the image data as an arc, the accuracy is improved.

Next, the subject is caused to wear the lens frame 1 (S20). Fig. 9 is a side view illustrating a state in which the lens frame according to this embodiment is being worn by a subject. As illustrated in Fig. 9, by causing the nose rest 432 of the nose rest supporting part 430 to come into contact with both sides of the nose N of the subject P and placing the curved parts 342A of one pair of the rear temple parts 34 on the ears EA of the subject P, the lens frame 1 can be worn by the subject P. In addition, at this time, the width of the one pair of temple members 30 is changed in accordance with the width of the face of the subject P. The width of the one pair of temple members 30 can be changed by adjusting the width of the bridge member 200 using the width adjusting mechanism 210 and adjusting the width of the nose rest frame 40 using the length adjusting mechanism 410. In addition, in accordance with an interpupillary distance PD of a subject, each lens housing 220 is moved in the horizontal direction using the lens gap adjusting mechanism 212 such that the reference point of the lens housing 220 coincides with the pupil center of the subject.

Next, as illustrated in Fig. 10, the lens frame 1 is adjusted such that the rotation center portion of the angle adjustable connection part 36 coincides with the rotation center point O of the image data (S22). More specifically, the length of the rear temple part 34 is adjusted by the length adjusting mechanism 344, the length of the nose rest frame 40 is adjusted by the length adjusting mechanism 410, and the angle of the nose rest frame 40 with respect to the rear temple part 34 is adjusted. In accordance with this, the position of the angle adjustable connection part 36 of the lens frame can be moved in the vertical plane of the forward/backward direction. The rotation center axis of the angle adjustable connection part 36 can be moved to coincide with the rotation center point O while referring to display of the display 56.

In addition, before mounting the lens frame 1, by performing adjustment of the length of the rear temple part 34, adjustment of the length of the nose rest frame 40, and adjustment of the angle of the nose rest frame 40 with respect to the rear temple part 34 in advance, only fine adjustment can be performed.

In this way, by performing Steps S20 and S22, the lens frame 1 can be worn by the subject P on the face such that the rotation center of the angle adjustable connection part 36 of the lens frame 1 is positioned on the lateral side of the rotation center point O of the subject P.

Next, the eye examination lenses L are mounted in the lens housing 220. A maximum of three eye examination lenses L are mounted in the lens housing 220. As the eye examination lenses, spherical power lenses, cylindrical power lenses, and prism lenses are appropriately combined and used. Then, the horizontal line HL is caused to coincide with the optical axis XL of the eye examination lens L, and the position of the eye examination lens L is adjusted such that a distance from the reference point CL on the rear face of the eye examination lens L to the corneal apex becomes VC (S24). In accordance with this, a distance from the reference point CL on the rear face of the eye examination lens to the rotation center point O becomes r = VC + CR. In addition, adjustment of the position of the eye examination lens L can be performed by rotating the front temple part 32 around the angle adjustable connection part 36 with respect to the rear temple part 34 and adjusting the length of the front temple part 32 using the length adjusting mechanism 324.

Next, eye examination data in the horizontal view is measured (S26). More specifically, a visual target T is presented at a viewing distance in which the subject needs spectacle lenses, for example, about 2 meters ahead, and the subject is caused to view the visual target T through the eye examination lenses in the horizontal view. Then, by interchanging the eye examination lenses on the basis of a response from the subject, the eye examination data in the horizontal view is measured while checking visual acuity or wearing comfort.

Next, as illustrated in Fig. 11, the front temple part 32 is rotated a designated angle below, for example, 15 degrees by the angle adjustable connection part 36 (S28). At this time, the lens housing 220 may be rotated to have a predetermined bending angle. Then, eye examination data in the downward view is measured (S30). More specifically, the visual target T is presented about 2 meters ahead of the subject and at a height positioned 15 degrees downward. Then, the subject is caused to view the visual target T through the eye examination lenses in a state in which his or her head is not moved. Then, by interchanging the eye examination lenses on the basis of a response from the subject, the visual acuity or the wearing comfort is checked, and eye examination data in the downward view is measured.

At this time, by rotating the front temple part 32 a predetermined angle below around the angle adjustable connection part 36 coinciding with the rotation center point O as the center, the relative position of the eye examination lens L and the subject's pupil can be maintained without being changed from that of the case of the horizontal view. In other words, the line of sight S of the subject coincides with the optical axis XL of the eye examination lens L, and the distance from the reference point CL on the rear face of the eye examination lens L to the corneal apex becomes VC.

When eye examination data in a downward view or a horizontal view is measured, by instructing a subject to view a visual target presented at a designated angle in a natural posture while allowing the subject to move his or her head and measuring a rotation angle of the head of the subject at that time by processing image data or the like, the front temple part 32 may be rotated downward by a difference between the designated angle and the rotation angle of the head by the angle adjustable connection part 36. Even in such a case, the line of sight S of the subject coincides with the optical axis XL of the eye examination lens L, and the distance from the reference point CL on the rear face of the eye examination lens L to the corneal apex becomes VC. The rotation angle of the head of the subject can be acquired using a front image or a side image of the subject or a measuring device such as a gyro sensor.

In accordance with the processes described above, the eye examination is completed.

The step of measuring eye examination data in the downward view and the step of measuring eye examination data in the horizontal view may be performed in reverse order.

On the basis of the eye examination data in the horizontal view, the eye examination data in the downward view, a difference between angles of the front temple parts at the time of an eye examination in the horizontal view and at the time of an eye examination in the downward view, and the like, the design of the spectacle lenses is performed (S32). More specifically, on the basis of the eye examination data and the angle, design information for the spectacle lens, which includes at least spherical power, cylindrical power, an astigmatism axis direction, a prism amount, and a prism base direction at a visual distance in which the subject needs the spectacle lens, is determined.

In accordance with the processes described above, the design of the spectacle lens is completed.

On the basis of the lens design information determined in this way, cutting of lens blanks, polishing, and the like are performed, and lenses that accurately reflect the required prism amount at the time of the downward view can be manufactured.

In addition, in the step of measuring the eye examination data in the horizontal view (S26) and the step of measuring the eye examination data in the downward view (S30), distances to the visual target presented to the subject may be, for example, set as about 5 meters and about 40 cm, and a progressive power lens also can be designed by measuring the eye examination data for distance vision and near vision.

At this time, when the eye examination data for near vision is measured, by instructing the subject to view a book or newspaper held in his or her hand in a natural posture and the examiner observing the position of the pupils of the subject from the front side of the lens frame 1, the angle of the front temple part 32 may be adjusted such that the pupils are positioned at the center of the eye examination lenses.

Then, on the basis of the eye examination data for distance vision, the eye examination data for near vision, a difference between the angles of the front temple parts at the time of eye examination for distance vision and at the time of eye examination for near vision, and the like, the design of the progressive power lens is performed (S32). More specifically, on the basis of the eye examination data and the angles, design information for the progressive power lens including at least spherical power (SPH: spherical power, CYL: cylindrical power, and AX: astigmatism axis direction), addition power (ADD: addition power), a corridor length, and the like for the distance vision is determined.

In accordance with the processes described above, the design of the progressive power lenses is completed.

Then, on the basis of the progressive design information determined in this way, cutting of lens blanks, polishing, and the like are performed, whereby the progressive power lens can be manufactured.

According to this embodiment, the following effects are mainly obtained.

According to the lens frame 1 of this embodiment, the temple member 30 includes each front temple part 32 supporting the lens holding member 20, the rear temple part 34 connected to a rear side of the front temple part 32, and the angle adjustable connection part 36 that is able to adjust an angle of the front temple part 32 with respect to the rear temple part 34 using a rotation center portion as a center, and the front temple part 32 has the length adjusting mechanism 324 that is able to adjust a length of the front temple part 32. In accordance with this, by mounting the lens frame such that the rotation center portion coincides with a rotation center point O and adjusting a length of the front temple part 32 using the length adjusting mechanism 324 in a horizontal view, a distance between the apex of the corneal C and the eye examination lens L is set to a predetermined distance VC, and an eye examination in the horizontal view can be performed. In addition, by rotating the front temple part downward around the rotation center portion as its center using the angle adjustable connection part 36, an eye examination in the downward view can be performed in a state in which the distance between the apex of the corneal C and the eye examination lens L is maintained to be the predetermined distance VC.

In addition, according to this embodiment, the rear temple part 34 has the length adjusting mechanism 344 that is able to adjust the length of the rear temple part 34. In accordance with this, the length of the rear temple part 34 can be adjusted, and the position of the rotation center portion can be easily changed.

In addition, according to this embodiment, the lens frame 1 has a lens gap adjusting mechanism that adjusts a distance between one pair of lens housings 220. In accordance with this, the reference point of the eye examination lens L held in the lens housing 220 can be adjusted to a desired position in accordance with the interpupillary distance PD of a subject.

In addition, according to this embodiment, the lens frame 1 includes a bending angle adjusting mechanism that adjusts the bending angle of the eye examination lenses L held in one pair of lens housings 220. In accordance with this, an eye examination can be performed in a state in which the bending angle of the eye examination lenses L held using the lens housings 220 is adjusted to match the lens frame.

In addition, according to this embodiment, the nose rest 432 that is connected to the temple member 30 and is configured to be able to adjust the position with respect to the rotation center portion is further included. According to such a configuration, the position of the rotation center portion can be adjusted in a state in which the lens frame 1 is worn by the subject on the face using the nose rest 432 and the temple member 30.

According to the lens frame 1 of this embodiment, the frame main body 10 is configured to be able to rotate the eye examination lenses L held in the lens housings 220 using the rotation center portion as its center in a state in which the distance between the rotation center portion and the eye examination lens L is maintained to be a predetermined distance, and thus the frame main body 10 can move the position of the rotation center portion forward/backward/upward/downward in a state in which the frame main body 10 is worn by the subject on the face.

In addition, according to this embodiment, the lens holding member 20 is connected to the front temple part 32 of the frame main body 10, and the length of the front temple part 32 is able to be adjusted. In accordance with this, the eye examination lens L can be maintained at a position for which the distance from the reference point CL on the rear face of the eye examination lens L to the corneal apex becomes the predetermined distance VC.

A method of measuring numerical values relating to eyesight according to this embodiment using the lens frame 1 described above includes: a mounting step of causing a subject to wear the lens frame 1 on the face and positioning the rotation center portion on a lateral side of the rotation center point of the subject (S20); an adjusting step of adjusting the length of the front temple part 32 such that a distance between the rotation center portion and a reference point of the eye examination lens L becomes a predetermined length CV (S24); a first measuring step of measuring a first numerical value (eye examination data in the horizontal view) relating to eyesight of the subject in a state in which the eye examination lens L is mounted in the lens housing 220 (S26); and a second measuring step of measuring a second numerical value (eye examination data in the downward view) relating to eyesight of the subject after rotating the lens housing 220 using the rotation center portion as center without changing the distance to the rotation center portion (S28) .

In accordance with this, eye examination data for distance vision and eye examination data for near vision can be measured without incurring an axial deviation while the distance between the eye examination lens L and the rotation center point O of the eyeball in the horizontal view and the downward view is maintained.

According to a method of designing spectacle lenses according to this embodiment, in order to measure eye examination data for distance vision in the horizontal view and measure eye examination data for near vision in the downward view as described above, when the design for the progressive power lens 500 including the near portion 520 used for viewing a near distance, the distance portion 510 for viewing a distance farther than the near distance, the middle portion 530, which has a progressive refraction function, disposed between the near portion 520 and the distance portion 510 is performed, it can be designed with high accuracy in accordance with an actual wearing state.

In this embodiment, although identification of the rotation center of the eyeball and position adjustment of the lens frame are performed using the frame position adjusting system 50, the present invention is not limited thereto. An ophthalmologist performing an eye examination may identify the rotation center of the eyeball of a subject by viewing a side face of the face of the subject though visual observation, and the position of the lens frame may be adjusted to match this.

In addition, in this embodiment, although the nose rest frame 40 is configured to be able to rotate around the angle adjustable connection part 36 as its center and to adjust the length using the length adjusting mechanism 410, the nose rest frame 40 does not necessarily need to be able to rotate and does not need to be able to be adjusted in length. For example, also by configuring the nose rest supporting part 430 to be a member that is able to support the lens frame 1 and is able to be deformed, the rotation center portion of the front temple part 32 can be configured to be able to freely move in a forward/backward direction and an upward/downward direction.

### [Reference Signs List]

- 1: Lens frame
- 10: Frame main body
- 20: Lens holding member
- 30: Temple member
- 32: Front temple part
- 34: Rear temple part
- 36: Angle adjustable connection part
- 40: Nose rest frame
- 50: Frame position adjusting system
- 52: Processing device
- 54: Imaging device
- 56: Display
- 58: Input device
- 200: Bridge member
- 202: First bending member
- 202A: Vertical part
- 202B: Horizontal part
- 204: Second horizontal member
- 210: Width adjusting mechanism
- 212: Lens gap adjusting mechanism (dial member)
- 214: Bending angle adjusting mechanism (dial member)
- 220: Lens housing
- 222: Lens housing main body
- 224: Pillar part
- 230: Connection part
- 320: First front temple member
- 322: Second front temple member
- 324: Length adjusting mechanism
- 340: First rear temple member
- 342: Second rear temple member
- 342A: Curved part
- 344: Length adjusting mechanism
- 400: First frame member
- 402: Second bending frame member
- 404: Third horizontal frame member
- 410: Length adjusting mechanism
- 420: Width adjusting mechanism
- 430: Nose rest supporting part
- 432: Nose rest
- 500: Progressive power lens
- 510: Distance portion
- 520: Near portion
- 530: Middle portion
- C: Corneal
- CL: Optical axis center
- E: Eyeball
- EA: Ear
- HL: Horizontal line
- L: Eye examination lens
- LE: Edge
- N: Nose
- O: Rotation center point
- P: Subject
- S: Line of sight
- T: Visual target
- UE: Upper eyelid edge
- XL: Optical axis

## Claims

1. A lens frame for eye examination comprising:
a lens holding member having one pair of lens holding parts each holding a lens for eye examination; and
one pair of temples,
wherein each of the temples includes:
a front temple part supporting the lens holding member;
a rear temple part connected to a rear side of the front temple part; and
an angle adjusting mechanism that is able to adjust an angle of the front temple part with respect to the rear temple part using a rotation center portion as a center, and
wherein the front temple part has a front length adjusting mechanism that is able to adjust a length of the front temple part.

2. The lens frame for eye examination according to claim 1, wherein the rear temple part has a rear length adjusting mechanism that is able to adjust a length of the rear temple part.

3. The lens frame for eye examination according to claim 1 or 2, further comprising a lens gap adjusting mechanism that is able to adjust a distance between the one pair of lens holding parts.

4. The lens frame for eye examination according to any one of claims 1 to 3, further comprising a bending angle adjusting mechanism that adjusts a bending angle of lenses held in the one pair of lens holding parts.

5. The lens frame for eye examination according to any one of claims 1 to 4, further comprising a nose rest that is connected to the temple and is configured to be able to adjust a position with respect to the rotation center portion.

6. A lens frame for eye examination comprising:
a lens holding member having one pair of lens holding parts each holding a lens for eye examination; and
a frame main body worn by a subject on the face,
wherein the frame main body is configured to be able to rotate the lenses held in the lens holding parts using a rotation center portion as center in a state in which a distance between the rotation center portion and the lenses is maintained at a predetermined distance, and
wherein the frame main body can move the position of the rotation center portion forward/backward/upward/downward in a state in which the frame main body is worn by the subject on the face.

7. The lens frame for eye examination according to claim 6,
wherein the lens holding member is connected to a front temple part of the frame main body, and
wherein a length of the front temple part is able to be adjusted.

8. A method of measuring numerical values relating to eyesight using the lens frame according to any one of claims 1 to 7, the method comprising:
a mounting step of causing a subject to wear the lens frame on the face and positioning the rotation center portion on a lateral side of a rotation center point of the subject;
an adjusting step of adjusting the lens frame such that a distance between the rotation center portion and a reference point of the lens becomes a predetermined length;
a first measuring step of measuring a first numerical value relating to eyesight of the subject in a state in which a lens for eye examination is mounted in the lens holding part; and
a second measuring step of measuring a second numerical value relating to eyesight of the subject after rotating the lens holding part using the rotation center portion as center without changing a distance to the rotation center portion.

9. A method of designing a spectacle lens, in which a spectacle lens is designed on the basis of the first numerical value and the second numerical value measured using the method according to claim 8.

10. The method of designing spectacle lenses according to claim 9,
wherein the spectacle lens is a progressive power lens including a near portion used for viewing a near distance, a distance portion used for viewing a far distance longer than the near distance, and a middle portion that is positioned between the near portion and the distance portion and has a progressive refraction function,
wherein a first numerical value relating to eyesight in distance vision is measured in one of the first measuring step and the second measuring step,
wherein a second numerical value relating to eyesight in near vision is measured in the other of the first measuring step and the second measuring step, and
wherein at least distance power, addition power, and a corridor length applied to the spectacle lenses are determined on the basis of the first numerical value and the second numerical value.
